# EUROPEAN PATENT APPLICATION

(11) **EP 1 921 445 A1**
(43) Date of publication of application: **14.05.2008**
(21) Application number: 06731539.0
(22) Date of filing: 11.04.2006
(51) Int. Cl.: G01N 27/416, G01N 27/406

(54) **HYDROGEN GAS SENSOR**

(30) Priority: 12.08.2005 JP 2005234522
(71) Applicant: Niigata TLO Corporation, Niigata-shi, Niigata 950-2181 (JP)
(72) Inventor: HARADA, Shuji, Niigata-shi Niigata, 9502156 (JP); MATSUDA, Kunio, Niigata-shi Niigata, 9502176 (JP)
(74) Representative: Schmitz, Hans-Werner
(86) International application number: PCT/JP2006/307592
(87) International publication number: WO 2007/020731

(57) **Abstract**

The object of the present invention is to provide a hydrogen gas sensor that may suffer less influences from the environmental space as well as exercise less influence to the environmental space. The hydrogen gas sensor 10 comprises a first electrode 12 and a second electrode14 are provided, and an electrolyte 16 disposed between the first electrode 12 and the second electrode 14. The first electrode and the second electrode are made of corresponding different materials in chemical potential for hydrogen gas. That is, the first electrode includes a relatively high chemical potential material, and the second electrode includes a relatively low chemical potential material. The hydrogen gas sensor detects hydrogen gas based on the electromotive force generated between these electrodes and, at least a contacting portion of the electrolyte with the first electrode and a contacting portion of the electrolyte with the second electrode are covered with a hydrogen selective permeable envelope 20.

## Description

### Field of the Invention:

This invention relates to a hydrogen gas sensor that is suitable for detecting hydrogen gas leaked in air or within gas pipings distributing other gases, or for analyzing the concentration of the leaked hydrogen gas.

### Background of the art:

It is desired in a future hydrogen energy utilizing society to establish a convenient hydrogen energy system from which the hazardous nature of hydrogen explosion is removed to develop the safety of the hydrogen energy system. It is required that the hydrogen gas sensor is configured to detect the hydrogen gas amount leaked in air or within the gas pipings distributing other gases instantaneously with high degree of accuracy, and to have an enhanced reliability. Particularly, with a hydrogen gas sensor used in a hydrogen leak alarm system, it is required that the hydrogen gas sensor is configured to detect the hydrogen gas concentration with high sensitivity within a low hydrogen concentration range below the explosion limit with a short detection time.

A conventional hydrogen gas sensor is configured on the detecting principles of semiconductor type, ionization type or combustion type, wherein the hydrogen amount is detected indirectly by utilizing the carrier concentration (semiconductor type), the ion concentration (ionization type) or the reaction heat (combustion type, or the hydrogen gas is burned to measure the vapor pressure), all of which are defined as an extensive physical value and converted into the corresponding electric value. With the conventional hydrogen gas sensor, therefore, it takes longer period of time to detect the hydrogen gas, e.g., by 100 seconds at the latest.

With the conventional (semiconductor type, ionization type or combustion type) hydrogen gas sensors, since the hydrogen gas concentration is detected by utilizing the carrier concentration, the ion concentration or the reaction heat as the hydrogen gas detection signals, it is necessary to provide a large detection area for a high sensitivity sensing. In this point of view, precision and sensitivity of the hydrogen gas sensor depend on the structure and the shape of the hydrogen sensor or the electrode size, so that the reduction in size of the hydrogen gas sensor is restricted. Moreover, the conventional (semiconductor type, ionization type or combustion type) hydrogen gas sensors have a deficiency to suffer from environmental gases. Particularly, when the hydrogen gas sensor is employed in the atmosphere containing gases such as gasoline, hydrocarbons and alcohols, which contain hydrogen elements, the hydrogen gas sensor may respond to those hydrogen-based gases, to thereby deteriorate the reliability of the hydrogen gas detection.

In this point of view, novel gas sensors based on electrochemical techniques have been developed and used, in addition to the above-mentioned conventional hydrogen gas sensors. These novel gas sensors can be classified as electromotive force measuring type hydrogen gas sensors and current detecting type hydrogen gas sensors. With the former type hydrogen gas sensors, as disclosed in Patent Publication No. 1 and 2, a hydrogen gas electrode is prepared as a standard or referential electrode having a hydrogen standard gas pressure atmosphere, and a detecting electrode is prepared as an operating electrode for measuring the gas to be detected (partial pressure of the hydrogen gas), wherein the difference in potential between these electrodes is measured as the output signal corresponding to the hydrogen gas concentration.

At the hydrogen electrode, atomic state hydrogen exists sufficiently on the electrode surface to form the standard potential of the electrode. Under the condition, when hydrogen gas contacts with the detecting electrode and is dissociated into atomic hydrogen, the detecting electrode exhibits an electric potential in proportion to the amount of the atomic hydrogen, and the difference in potential between the hydrogen gas electrode and the detecting electrode is detected proportionally with a function of the hydrogen gas concentration. In other words, with the new hydrogen gas sensors, since the detecting hydrogen gas pressure is measured in comparison with the standard hydrogen gas pressure, both of electrodes must be separated and isolated in the standard hydrogen gas atmosphere and the detecting gas atmosphere respectively, so that another "standard hydrogen gas pressure room must be provided in addition to the detecting gas pressure room. Also, this necessitates the hydrogen gas sensors to have a certain dimension for containing the standard hydrogen gas pressure room, and the use conditions of the hydrogen gas sensors are limited.

On the other hands, with the current detecting type hydrogen gas sensors, the current value is classified as an extensive physical value, so that in order to realize a high precise measurement using the hydrogen gas sensors, the areas or the volumes of the hydrogen gas sensors must be enlarged and external power supplies are necessary to provide the hydrogen gas sensors with electric current.

In the situation described above, the inventors have developed a novel hydrogen gas sensor that utilizes two electrodes respectively made of different materials having different chemical potentials relative to hydrogen gas, wherein the first electrode contains a material with a relatively high chemical potential to function as a detecting electrode for a hydrogen gas, and the second electrode contains a material with a relatively low chemical potential to function as a standard or referential electrode for a hydrogen gas. This hydrogen gas sensor can be simplified in configuration and reduced in size, because this sensor can be dispensed with the hydrogen gas chamber that is inevitable to those conventional electromotive force measuring type hydrogen gas sensors. This sensor also can detect the hydrogen gas instantaneously, because the sensor detects the hydrogen concentration based on chemical potentials.

[Patent Publication No. 1] Japanese Patent Application Laid-open No. 2003-270200
[Patent Publication No. 2] Japanese Patent Application Examined Publication No. 5-663

### Disclosure of the Invention:

### Problem to be solved by the Invention:

As described above, the hydrogen gas sensor utilizing two electrodes made of different materials with different chemical potentials relative to hydrogen gas can be used in detection spaces while being contained in a case providing the sensor with a reinforcing or explosion-proof function. However, if the sensor is used in a casing having a structure open to the environment, the sensor may suffer from influences of various gases (oxygen in particular) or moisture existing in the atmosphere, which may cause troubles when performing a quantitative measurement of the hydrogen gas. Also, such an open-to-environment type sensor may affect the detection space with contaminations. On the other hand, if the sensor is used in a semi-sealed condition, it may suffer from residual hydrogen gas problem.

It is an object of the present invention to provide a hydrogen gas sensor that may suffer less influence from the environmental space as well as exercise less influence to the environmental space.

### Means for solving the Problem:

In order to achieve the object, a hydrogen gas sensor defined in Claim 1 comprises a first electrode, a second electrode and an electrolyte contacting with the first electrode and the second electrode, wherein the first electrode and the second electrode are made of corresponding different materials in chemical potential for hydrogen gas, and the first electrode is made of higher chemical potential material and the second electrode is made of lower chemical potential material, wherein the hydrogen gas is detected on an electromotive force generated between the first electrode and the second electrode, and wherein at least a contacting portion of said electrolyte with said first electrode and a contacting portion of said electrolyte with said second electrode are covered with a hydrogen selective permeable envelope.

The invention defined in claim 2 is a hydrogen gas sensor as defined in claim 1 wherein residual hydrogen gas within said envelope is reacted by temporary short-circuiting said pair of electrodes.
The invention defined in claim 3 is the hydrogen gas sensor as defined in claim 1 or 2, wherein said envelope is configured by superimposing multiple different materials having different barrier properties.

The invention defined in claim 4 is the hydrogen gas sensor as defined in any one of claims 1-3, further comprising a device for adjusting temperature of the space within said envelope.

The invention defined in claim 5 is the hydrogen gas sensor as defined in any one of claims 1-4, wherein said first electrode is made of a hydrogen selective permeable metal.

According to the invention defined in claim 5, the first electrode (detecting electrode) is formed as a metal film to function as a hydrogen selective permeable film, whose one surface is facing to a detection space and the other facing to the second electrode (standard electrode) via the electrolyte. The metal film is provided with gas barrier properties against gases other than hydrogen, and capable of concurrently detecting hydrogen chemical potential value on the surface after hydrogen has been transmitted therethrough as a voltage difference between the second electrode. The sensor can also be used by covering the area other the hydrogen selective permeable metal film.

The invention defined in claim 6 is a hydrogen gas leak alarm system comprising a hydrogen gas sensor as defined in any one of claims 1-5 and a voltage comparator, wherein an electromotive force variation as a hydrogen gas detecting information from said hydrogen gas sensor is compared with a reference voltage of said voltage comparator, thereby to put out an signal on the comparison of said electromotive force variation and said reference voltage.

The invention defined in claim 5 is the hydrogen gas sensor as defined in any one of claims 1-4, wherein said first electrode is made of a hydrogen selective permeable metal. The invention defined in claim 7 is the hydrogen gas leak alarm system as defined in claim 6, wherein said voltage comparator is configured such that a threshold voltage of a Schmitt inverter is defined as said reference voltage, and compared with an input voltage corresponding to said hydrogen gas detecting information, thereby to put out said signal.

The invention defined in claim 8 is a hydrogen gas sensor array comprising a plurality of hydrogen gas sensors as defined in any one of claims 1-5, wherein said hydrogen gas sensors are arranged on the same substrate.

The invention defined in claim 9 is a hydrogen gas analyzer comprising a hydrogen gas sensor as defined in any one of claims 1-5 and an electric circuit for detecting an electromotive force from said hydrogen gas sensor, wherein hydrogen gas concentration is detected in dependence on the intensity of said electromotive force.

The invention defined in claim 10 is a hydrogen gas sensor element comprising a hydrogen gas sensor as defined in any one of claims 1-5 and a photo sensor for detecting hydrogen gas shielding contamination from external environment through the detection of an optical signal from an external LED, whereby Fail-Safe function for enhancing reliability in hydrogen gas detection is applied to said hydrogen gas sensor element.

### Effects of The Invention:

According to the invention defined in claims 1-5, a hydrogen gas sensor can be achieved that may suffer less influence from the environmental space as well as exercise less influence to the environmental space so that it can perform a stable detecting function.

According to the invention defined in claims 6-10, a hydrogen gas leak alarm system, a hydrogen gas sensor array, a hydrogen gas analyzer, or a hydrogen gas sensor element are achieved which utilizes the advantageous hydrogen gas sensor defined in claims 1-5.

### Best Mode for Carrying out the Invention:

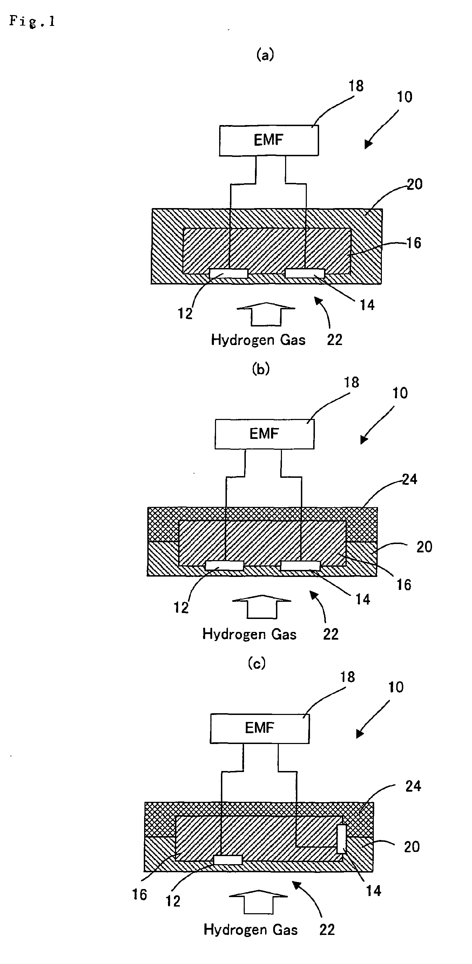

An embodiment of the present invention is described hereinafter. FIG. 1 is a view illustrating a theoretical aspect of the structure of the hydrogen gas sensor according to the present invention. The hydrogen gas sensor 10 comprises a pair of electrodes 12, 14, a solid or liquid electrolyte contacting with both of the electrodes 12, 14, and an electromotive force measuring device 18 connected between the electrodes 12, 14. This sensor 10 is clad with an envelope 20 made of a material having a low hydrogen barrier property or a selective permeability for hydrogen. The first electrode 12 functions as a detecting electrode for hydrogen gas, and the chemical potential is remarkably varied when the hydrogen gas contacts with the first electrode 12. The second electrode 14 functions as a standard electrode for the hydrogen gas, and the chemical potential of the second electrode 14 is almost unvaried or if varied, the variable degree is very small when the hydrogen gas contacts with the second electrode 14.

The portion of the hydrogen permeable envelope 20 covering the detecting electrode 12 constitutes a hydrogen gas detecting surface 22. Though the hydrogen permeable envelope 20 can be formed to cover the whole sensor 10 as shown in FIG. 1(a), it is permissible as long as it covers at least the portion where the detecting electrode and the standard electrode are contacting. As shown in FIG. 1(b), the portions of the detecting electrode, the standard electrode and the electrolyte, which are not covered with the hydrogen permeable envelope 20 is covered with a sealing portion 24 basically made of a gas tight material. Thus, the electrolyte 16 or the electrodes 12, 14 basically do not contact with the outer gases or liquids. As shown in FIG. 1(c), the standard electrode 14 does not necessarily have to stay on the detection surface 22.

The first electrode 12 is made of a first electrode material of higher chemical potential relative to hydrogen such as Pt, Pt alloy, Pd, Pd alloy which are higher absorption-dissociation active degree materials for the hydrogen gas. The first electrode 12 can be made of the above-exemplified materials as they are, or as they are supported on a given substrate. The first electrode 12 can be formed in any construction within a scope of the present invention only if the first electrode 12 can function as the detecting electrode for hydrogen gas.

The second electrode 14 is made of a second electrode material of lower chemical potential such as Ni, Ni alloy, Ti, Ti alloy, Cu, Cu alloy, Fe, Fe alloy, Al, Al alloy and organic conductive material which are lower absorption-dissociation active degree materials. The second electrode 14 can be made of the above-exemplified material, but can be formed in any construction within a scope of the present invention only if the second electrode 14 can function as the standard electrode for the hydrogen gas. These first and second electrodes 14 can be formed into various shapes such as plates, wires, pipes, discs, or rectangles.

The electrolyte 16 may be made of any convenient substances of solid or liquid materials, and among others, solid electrolyte such as phosphorous tungstic acid are advantageous due to its easy handling ability and stable performances. Phosphorous tungstic acid is also advantageous in having higher adhesion for the aforementioned first electrode 12 and second electrode 14. The solid electrolyte 16 may contain reinforcing materials such as glass wool in addition to the electrolyte such as phosphorous tungstic acid. In this case, not only the strength of the solid electrolyte 16 can be enhanced, but also the adhesion of the solid electrolyte 16 to the first electrode 12 and the second electrode 14 can be further enhanced.

The phosphorous tungstic acid and the phosphorous molybdic acid is normally obtained in the form of powder, so that in the fabrication of the solid electrolyte, the powdery phosphorous tungstic acid or phosphorous molybdic acid is solidified by compression molding into a pellet shape. However, the solid material thus compression molded is too fragile to be employed for a long-term use as solid electrolyte as it is. Therefore, it is preferable to add some glass wool into a resolved phosphorous tungstic acid material, which is a solution of the powdery phosphorous tungstic acid in a given solvent (such as ion exchanged water), and to solidify it into a solid electrolyte. The manufacturing process of the solid electrolyte is described hereinafter. This process is also effective in enhancing the adhesion between the electrodes and the solid electrolyte..
(1) A powdery raw material for the intended solid electrolyte (such as phosphorous tungstic acid) is melted in a given solvent to be liquidized,
(2) Reinforcing materials are put in a space within a mold for forming the solid electrolyte, and electrodes are assembled therein,
(3) The liquidized raw material is flowed into the mold containing the reinforcing materials,
(4) The liquidized raw material is solidified to form the solid electrolyte as the primitive form of the hydrogen gas sensor.

The envelope 20 is preferably permeable to hydrogen and impermeable to other unwanted or unfavorable gas or liquid components such as water vapor or oxygen. Hydrogen is an element having a small molecular size and the lightest mass, and hydrogen molecule is non -polar. Therefore, commonly used resins are available if they are formed to have a certain thickness to exhibit such a property because they have larger permeability to hydrogen than to vapor or oxygen. It is also available to use some of functional resins that have been developed for various uses. For example, various resins have been developed as wrapping films or gas permeation membranes, which are impermeable to oxygen. It is also possible to use a laminated film, which is manufactured by superimposing multiple layers of different materials so as to provide a selective permeability for hydrogen.

The barrier property against vapor or oxygen (hereinafter called as "gas barrier property") generally decreases as temperature rises. Thus, it is desirable to use a material that can preserve the gas barrier property at a temperature of an environment where the sensors are supposed to operate. It is also desirable to select a resin that can exhibit stable hydrogen permeability in a high relative humidity environment, because the resin is used while contacting with the electrolyte. Other general property such as superior heat resistance is also deemed to be necessary as well as the material is suited for the manufacturing process such as injection molding.

Here are some examples of the resins that can maintain gas barrier property in a high relative humidity environment: PET (polyether terephthalate), PEI (polyether imide), PVDC (polyvinylidene chloride (saran resin: trademark)), liquid crystal polyether (VECTRA: trademark). Among others, PEI or liquid crystal has a low transmission factor and superior heat resistance, and is suited to a compression molding process so as to be better adapted to a mass production system. It is also possible to apply a multilayer molding process in case of using the injection molding process.

With such an arrangement of electrodes 12, 14 and electrolyte 16, the sensor has a function of detecting the exterior hydrogen gas. That is, since the first electrode 12 and the second electrode 14 are made of different materials having different chemical potential relative to hydrogen gas, existence of the hydrogen gas within the envelope can be detected as a chemical potential difference between hydrogen on both of the electrodes, i.e., an electromotive force difference. Meanwhile, the electrodes 12, 14 and electrolyte 16 are isolated from the outer space with regard to vapor or oxygen, so that the fluctuation of output signals from the sensor resulting from variation in hydration concentration in the solid electrolyte or variation in oxygen concentration in an atmospheric air can be suppressed.

Since the hydrogen gas sensor 10 is so constructed that it is isolated from the environment except for allowing transmission of hydrogen, so that the sensor itself does not exercise any influences to the space where the sensor is located. Therefore, it can be placed in any space necessary to maintain its delicate atmosphere such as the space within various industrial ductworks or clean rooms without anxiety.

Since the hydrogen gas sensor 10 detects the existence of hydrogen as electromotive force differences, it has an extremely high detection speed and exhibits a high detection performance at low hydrogen concentration ranges. Since the sensor can be manufactured only by assembling electrodes 12, 14 and electrolyte 16 within one envelope 20 so that the standard hydrogen gas pressure chamber is not necessary, the structure can be simpler and size can be smaller.

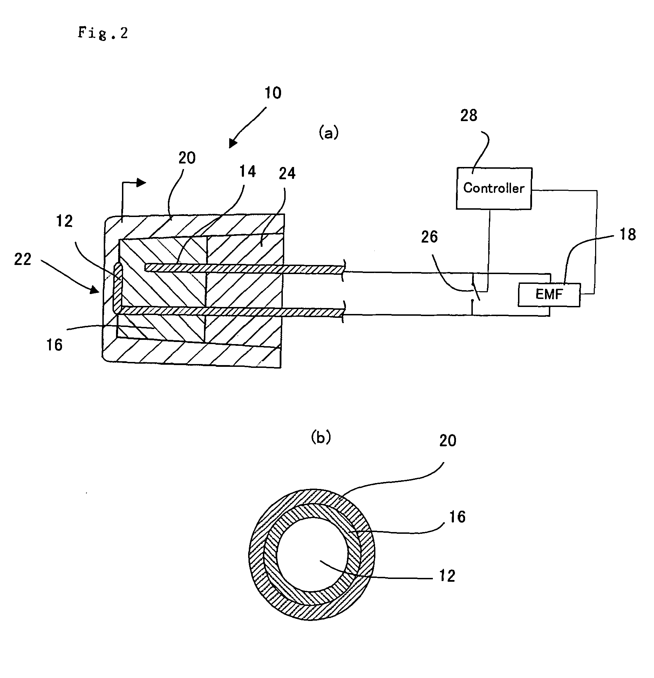

FIG. 2 depicts an embodiment of the invention in detail in a manner to correspond to FIG. 1(c). The hydrogen gas sensor 10 is generally formed in a cylindrical or truncated cone shape with a size of approximately 6.0mmφ×6.0mm, although the invention is not limited thereto in shape or in size. The envelope 20 of this embodiment is made of PET configured in a cylindrical shape with a 0.5 mm thick bottom portion forming a detection surface22.

At the rear side of the detection surface 22, a disc-shaped detecting electrode 12 made of a material having a higher chemical potential (Pt in the embodiment) is attached and a lead wire made of the same material is drawn therefrom. The inner space of the envelope 20 is filled with the electrolyte 16 in which a standard electrode 14 made of a relatively low chemical potential (Ni in the embodiment) is embedded. The opening end of the envelope 20 is filled with a sealant (sealing portion) made of a gas tight material such as resin so as to seal the electrolyte 16 and the electrodes 12, 14 within the envelope 20. Although NaCl solution is used as the electrolyte 16 because this embodiment is a trial product, it is desirable to use a solid electrolyte for the practical use products due to endurance or mass productivity reasons.

The hydrogen gas sensor 10 is manufactured by injecting resin material into a mold. In order to apply to a mass production system, so-called insert molding process is conveniently used, wherein metal elements such as electrodes are pre-assembled in the mold, into which the resin is filled and solidified. The manufacturing process can be selected properly depending on the materials for the envelope, sealant or electrolyte.

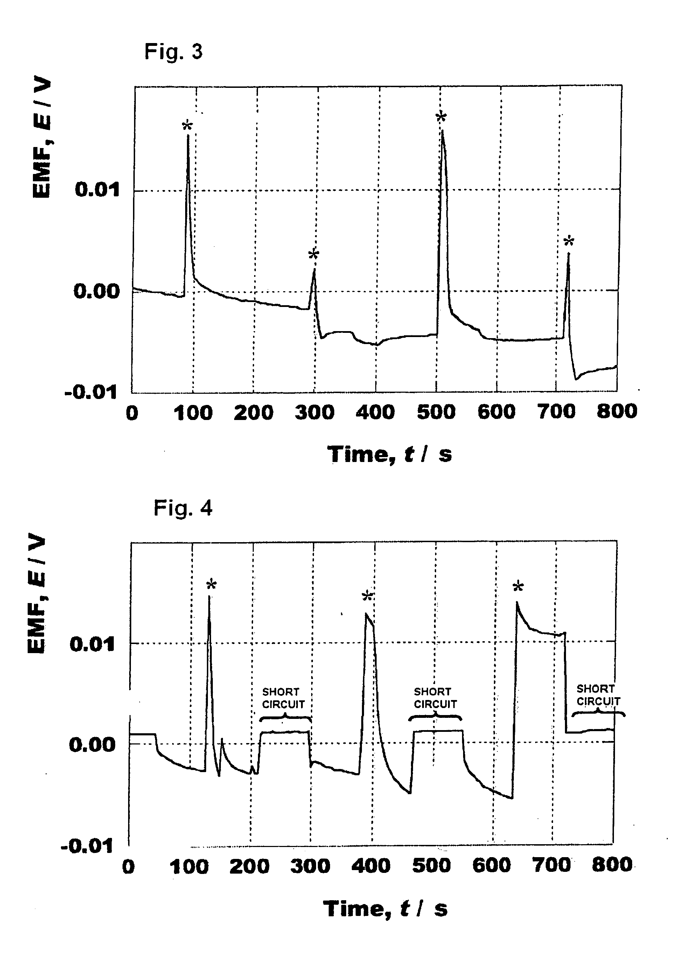

FIG. 3 shows the result of a hydrogen detecting test using the hydrogen gas sensor 10 shown in FIG. 2. The test is conducted by blowing hydrogen gas from a hydrogen container towards the hydrogen gas sensor at a timing shown with an asterisk in the chart repeatedly with a certain interval, and measuring the electromotive force generated between the electrodes. It is apparent that the measurement shows a peak electromotive force simultaneously with the blowing of hydrogen to indicate that the hydrogen detection is accomplished. However, the result also shows that a repetition of hydrogen blow with a small interval will result in an inadequately intensive peak. This is likely that a dead time is generated, during which hydrogen is not detected, after each measurement, because the sensor is a sealed type so that the hydrogen once taken in the sensor is not easily evacuated so that the sensor is in a state of continuously detecting the hydrogen gas.

In order to overcome the above-mentioned problem, the sensor of this embodiment is provided with a short-circuit switch 26 and a controller 28 for opening/closing the switch at a proper timing. When the detecting electrode 12 and the standard electrode 14 are short-circuited, current runs between the electrodes so that hydrogen and oxygen react at the standard electrode 14 to generate water and remove the residual hydrogen within the sensor. In this embodiment, the output signal of the electromotive force measuring device 18 is input to the controller 28 so that the controller controls the short-circuit switch 26 to close it immediately after verifying the electromotive force peak.

FIG. 4 shows the test result by activating the short-circuit switch 26. The graph shows that the application of the short-circuit switch has overcome the dead time problem. In the graph, asterisk (*) shows the timing when hydrogen gas is blown onto the sensor, and the sensor seems to respond to the blow of hydrogen. Although the controller controls the short-circuit switch immediately after verifying the electromotive force peak in this embodiment, the controller may control the switch in a way to repeat short-circuiting at a proper interval. Hydrogen having entered into the electrolyte 16 is in a thermal equilibrium state with exterior hydrogen through the hydrogen permeable film, and the short-circuit process removes the hydrogen gas and renders the current within the circuit zero naturally. This process consumes also oxygen and generates water, so that it is necessary to provide other devices for properly providing oxygen gas and removing generated water, in order to use this function continuously.

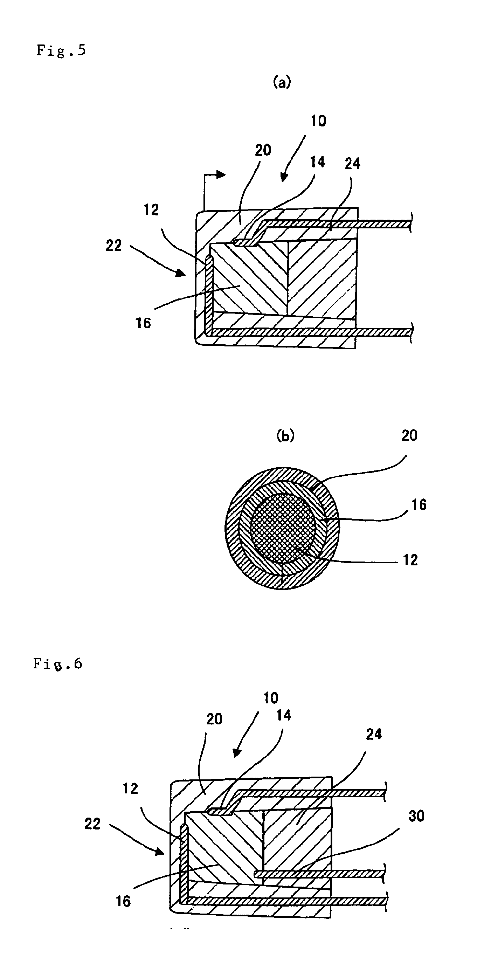

FIG. 5 shows a modified embodiment of the hydrogen gas sensor shown in FIG. 2, in which the detecting electrode 12 is formed as a mesh so that the boundary surface between the detecting electrode 12 and the electrolyte 16 is enhanced. Since the boundary surface between the detecting electrode 12 and the electrolyte 16 is a site for transforming hydrogen gas into hydrogen ions, the enhancement of the effective area of the boundary surface can enhance the sensitivity of the hydrogen sensor. The mesh type electrode will also serve to reinforce the structure of the sensor so as to avoid the deformation of the detection portion due to pressure difference, and is particularly advantageous if the sensor is located in a piping causing pressure difference between the exterior and interior of the sensor. In order to enhance the effective area, any suitable means other than the mesh electrode is applicable such as to use an electrode made of porous material. In the embodiment, the electrodes and the lead wire is not embedded in the electrolyte 16, and the electrode is arranged at the surface and the lead wire is guided through the envelope 20. This configuration reduces the influence caused by the residual hydrogen within the electrolyte 16. Further, this embodiment adopts plural lead wires arranged in the envelope to reinforce the sensor structure. In order to obtain the same effect, the lead wire may be made of a cylindrically formed mesh.

FIG. 6 shows a modified embodiment of the hydrogen gas sensor shown in FIG. 5, in which an operative electrode 30 is additionally provided. In the embodiment, by flowing current between the operative electrode 30 and the detecting electrode 12 to generate oxygen on the surface of the detecting electrode 12 so that the potential difference between the standard electrode 14 and the detecting electrode 12 is adjustable. With this configuration, the sensor can forcibly remove the hydrogen adsorbed on the detecting electrode 12 by self-discharging instead of supplying current from outer sources. Therefore, the sensor can be provided with a reset function with which the boundary surface of the detecting electrode 12 is set back to a predetermined standard value so as to restore the sensor's hydrogen detecting performance to its initial level.

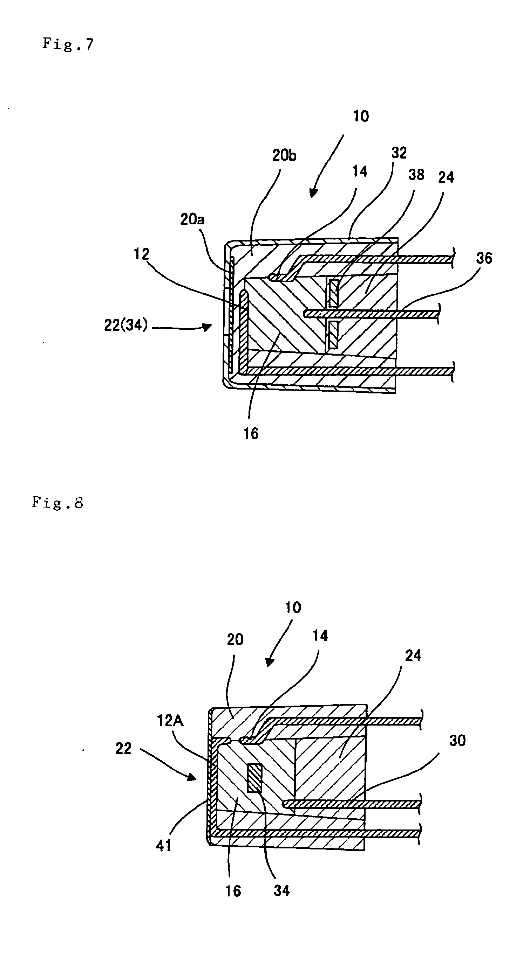

FIG. 7 shows another modification of the hydrogen gas sensor shown in FIG. 5, in which a reinforcing casing 32 made of metal, e.g., is provided as an outermost covering. This reinforcing casing 32 does not allow transmission of hydrogen or other gases, or liquids. This casing 32 is formed with a window 34 at a location corresponding to the detecting surface 22. A first envelope 20a made of a resin, which is permeable to hydrogen but exhibits a high barrier property against other gases including oxygen, is provided to cover the window 34. Also, the inner surface of the first envelope 20a is covered by a second envelope 20b made of a resin, which is permeable to hydrogen but exhibits a high barrier property against water vapor, on which a sensor portion of a similar structure shown in FIG. 5 is assembled. This embodiment is further provided with a heater 36 extending into the electrolyte 16, to which a controlled amount of electricity is provided depending on an output signal from a temperature sensor (not shown), so that the sensor temperature is maintained at a predetermined operative range.

The sensor of this embodiment exhibits higher sealing ability compared to other embodiment mentioned above as well as higher structural strength. Therefore, the sensor suffers less influence from the environment to perform accurate detection for a longer period. Since the sensor exercise less influence to the environmental space vice versa, it can be placed at any necessary locations without anxiety. Since the sensor is provided temperature adjustment function, it can perform accurate detection under a low temperature condition in which usual sensor is not supposed to work. The sensor of this embodiment is also provided with an absorbing agent 38 for absorbing water, which is generated in the short-circuiting process described above, because the sensor structure is so airtight that the generated water is not easily evacuated. The sensor may also be provided with a proper type of oxygen source that is necessary for removing loaded hydrogen.

FIG. 8 illustrates a hydrogen gas senor according to another embodiment of the invention. Here, the detecting electrode 12A is made of a metal thin film such as Pd or Pd alloy and also serves as a hydrogen selective permeable film. Pd (palladium) has been put in practical use as a purification film for hydrogen, and is applied for this purpose at a higher temperature in order to exhibit a higher hydrogen diffusion rate. It is important for this sensor not to form a hydride between the metal film and the hydrogen, so that the metal film should be made from a material having enough hydrogen diffusion rate as well as less hydride formation tendency at lower temperatures. The sensor also is provided with a heater 36 to raise the hydrogen diffusion rate.

Hydrogen having entered into the electrolyte 16 is in a thermal equilibrium state with exterior hydrogen through the metal film. In the embodiment, an operative electrode 30 is provided to forcibly remove the hydrogen adsorbed on the detecting electrode 12A in a similar manner as the embodiment shown in FIG. 6. A protective film 41 is also provided, which is a film or mesh made of a hydrogen selective permeable material, for protecting the detecting electrode 12A. By providing a short-circuit switch 26 shown in FIG. 2 and short-circuiting the two electrodes, the trapped hydrogen gas is converted into water by consuming oxygen within the sensor. In order to supply necessary oxygen and remove generated water, the sealing portion 24 may be made from oxygen/water permeable material, so that the short-circuiting operation can continuously work.

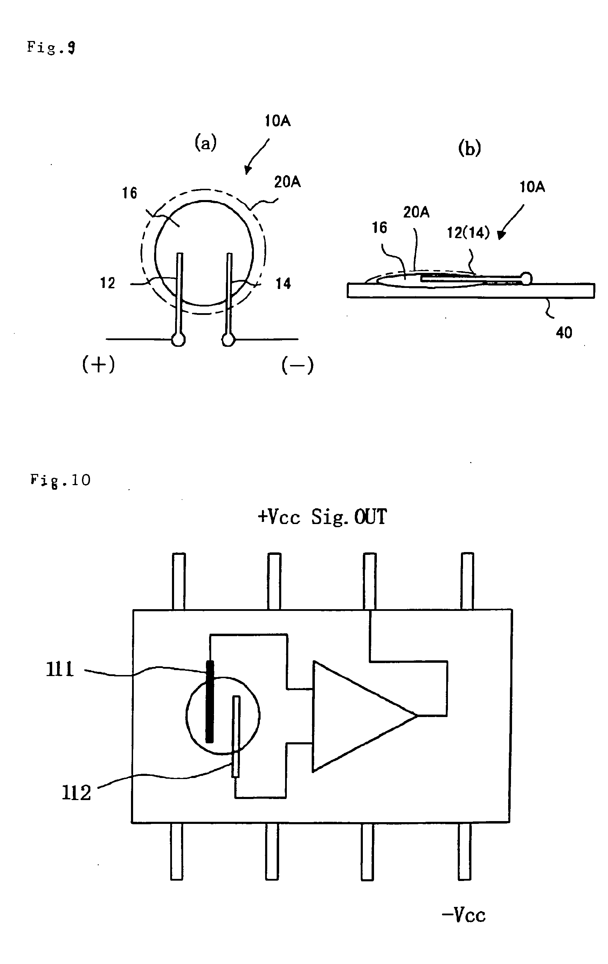

FIG. 9 illustrates another embodiment of the invention, in which the hydrogen gas senor 10A is generally formed in a plate shape. Specifically, a solid electrolyte film 16 is formed on an insulating substrate 40, and a first electrode 12 and a second electrode 14 are respectively formed at a distance from each other on the solid electrolyte film 16. Further, an outer coating film 20A that has selective hydrogen permeability as well as barrier properties against other gases or liquids is formed on the electrolyte film 16 and electrodes 12, 14. The outer coating film 20A is formed to cover at least boundary portions of the electrolyte film 16 and electrodes 12, 14.

These electrolyte film 16, electrodes 12, 14 and the outer coating film 20A can be formed by using a film deposition or etching process utilized in a semiconductor manufacturing system so that a small sized sensors are produced in a large amount. In this case, a number of sensors are formed on one substrate so that each piece of sensor can be obtained by dicing the substrate. In this manufacturing process, measuring circuits, the short-circuiting circuit illustrated in FIG. 2, or control circuits can be integrally formed simultaneously. In a similar manner, the sensor can be assembled as a part of an integrated circuit having other functions. FIG. 10 is a schematic diagram illustrating the sensor shown in FIG. 9 integrated in a electric circuit. The detecting electrode 111 and the standard electrode 112 is arranged to extend from oppose directions.

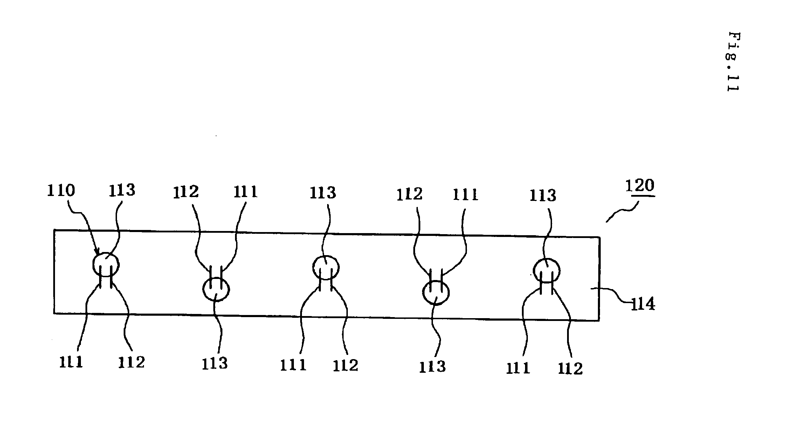

FIG. 11 is a structural view illustrating a hydrogen gas sensor array according to the present invention. In the hydrogen gas sensor array illustrated in FIG. 11, a plurality of hydrogen gas sensors according to FIG. 4 are arranged on an insulating substrate 114. In this case, since each hydrogen gas sensor 110 can detect hydrogen gas, the array can detect the hydrogen gas depending on the detecting position. Therefore, the array is suitable for hydrogen gas leak in the wide area such as a hydrogen gas station.

If the hydrogen gas sensors are arranged in high density, the array can be constituted as a leak detector using each hydrogen gas sensor as a probe.

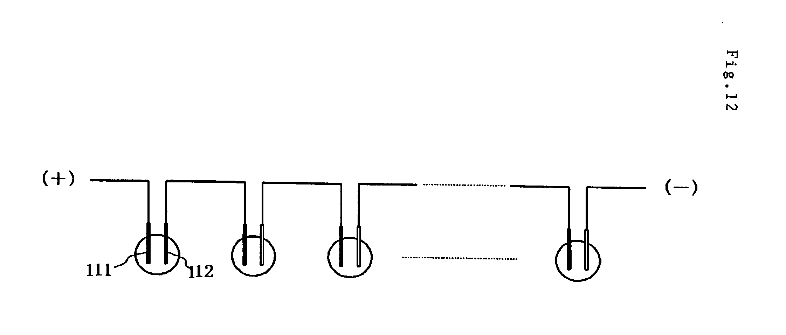

In the array illustrated in FIG. 11, when the hydrogen gas sensors are connected to one another in series as illustrated in FIG. 12, the electromotive forces from the hydrogen gas sensors are added up to provide larger detecting voltage.

The hydrogen gas sensor illustrated in FIGS. 1 and 9 or the hydrogen gas sensor array illustrated in FIGS. 11 and 12 can be installed in a suitable electric circuit, and the detecting voltage is detected via the electric circuit. If the hydrogen gas sensor is installed into the electric circuit, the electromotive force of the hydrogen gas sensor becomes constant under non-hydrogen atmosphere, which is defined as an electrostatic potential between the first electrode and the second electrode. In this case, therefore, if the electromotive force is measured via the electric circuit, the operating reliability of the hydrogen gas sensor can be appropriately confirmed, so that the hydrogen gas sensor can have the self-diagnosed function.

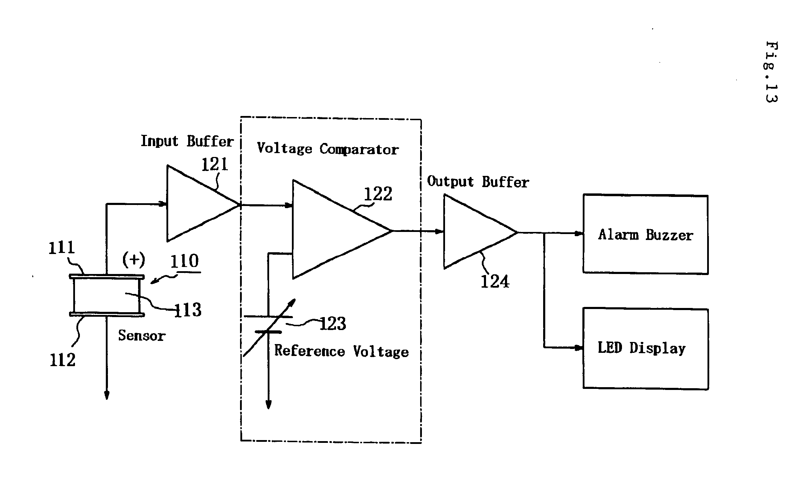

FIGS. 13-15 are block diagrams of a hydrogen gas leak alarm system, a hydrogen gas leak controlling system and a hydrogen gas leak transmitting system that utilizes hydrogen gas sensors according to the present invention.
FIG. 13 is a block diagram of the hydrogen gas leak alarm system utilizing the hydrogen gas sensor of the present invention. The electromotive force variation as a hydrogen gas detecting information from the hydrogen gas sensor 110 is input into an input buffer 121 of high input impedance, converted in impedance and signal level, and input into a voltage comparator 122. In the voltage comparator 122, the input signal is compared with the reference voltage of a standard power supply 23, and the thus obtained compared result is output via an output buffer 124 provided at the next stage, and input into an alarm buzzer or a light-emitting diode panel (not shown), thereby constituting the hydrogen gas leak alarm system.

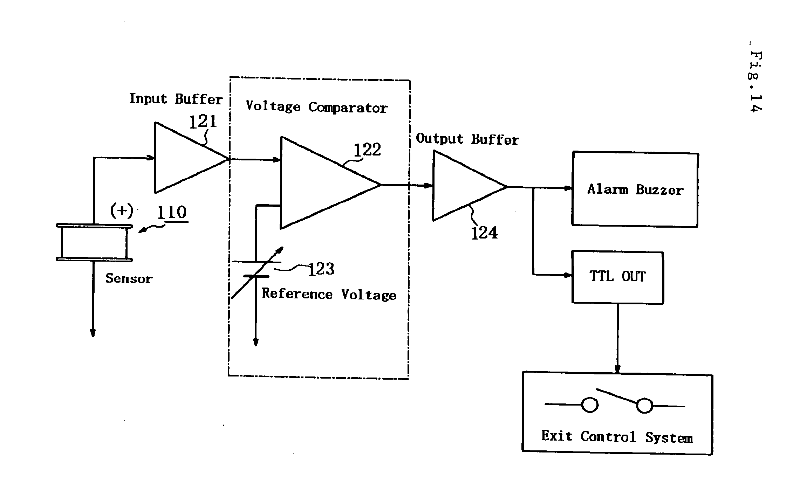

FIG. 14 is a block diagram of the hydrogen gas leak controlling system utilizing the hydrogen gas sensor of the present invention. In the system, when hydrogen gas over a predetermined level is detected by the hydrogen gas sensor, the hydrogen gas leak information is known via a light-emitting diode panel and at the same time, an external relay or magnetic valve is operated.

The electromotive force variation as a hydrogen gas detecting information from the hydrogen gas sensor 110 is input into an input buffer 121 of high input impedance, converted in impedance and signal level, and input into a voltage comparator 122. In the voltage comparator 122, the input signal is compared with the reference voltage of a standard power supply 123, and the thus obtained compared result is output via an output buffer 124 provided at the next stage, and input into an alarm buzzer for warning hydrogen gas leak, a light-emitting diode panel (not shown) for displaying the hydrogen gas leak or an Exit control System for operating an external relay or magnetic valve via a TTL OUT, thereby constituting the hydrogen gas leak alarm system.

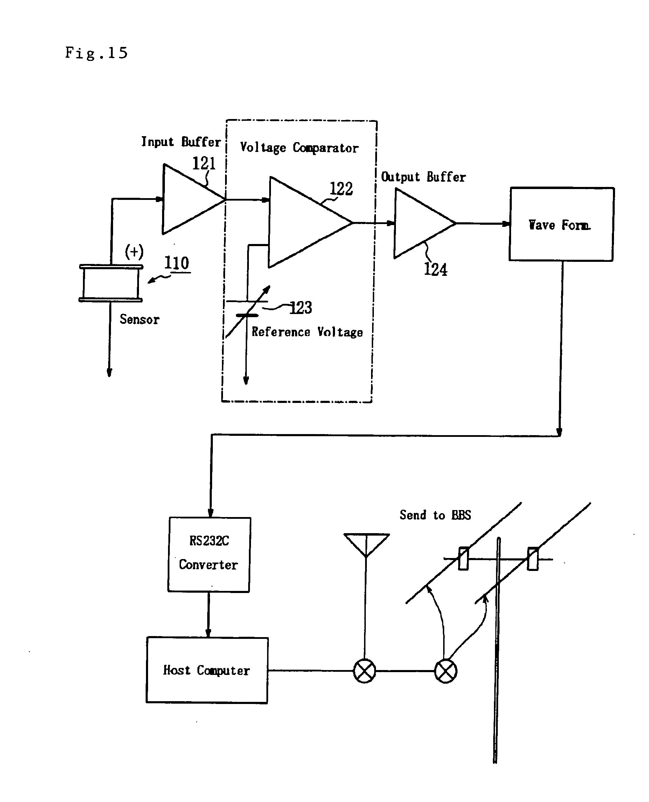

FIG. 15 is a block diagram of the hydrogen gas leak transmitting system utilizing the hydrogen gas sensor of the present invention. In the system, when hydrogen gas over a predetermined level is detected by the hydrogen gas sensor, the hydrogen gas leak information is transmitted to a local area via a wireless LAN or a BBS by using a computer.

The electromotive force variation as a hydrogen gas detecting information from the hydrogen gas sensor 110 is input into an input buffer 121 of high input impedance, converted in impedance and signal level, and input into a voltage comparator 122. In the voltage comparator 122, the input signal is compared with the reference voltage of a standard power supply 123. The thus obtained compared result is output via an output buffer 24 provided at the next stage, converted in signal level (Wave Form), transmitted to a host computer via an RS232C port and the like as a typical serial communication of PC, and transmitted to a local area via a wireless LAN or a BBS.

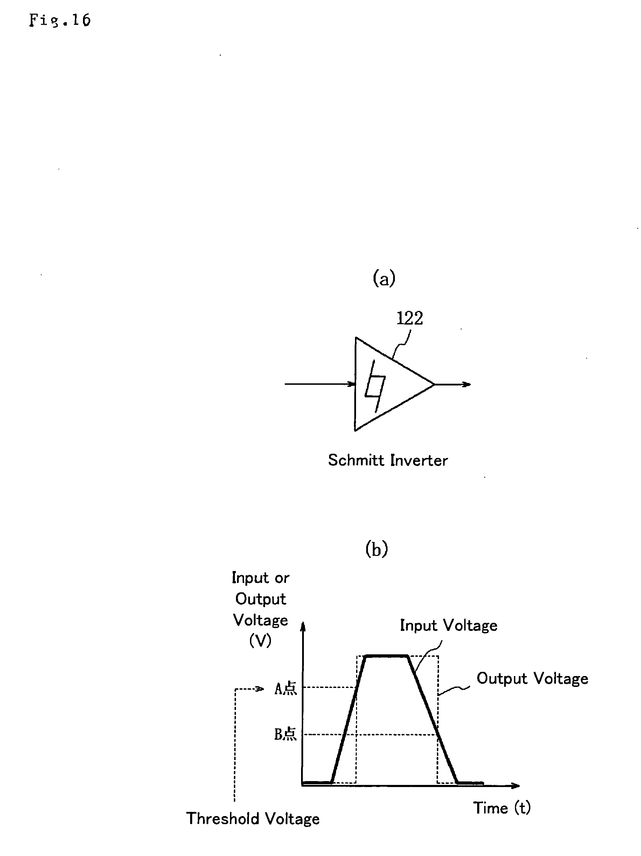

FIG. 16 is a schematic explanatory view of the structure and operation of the voltage comparator in the systems illustrated in FIGS.13-15. The voltage comparator 122 is a most important component among all of the components of the systems. When a voltage over the reference voltage is output from the input buffer 121 and input into the voltage comparator 122, the output voltage of the voltage comparator 122, which is almost equal to the power supply voltage, is on, and when a voltage below the reference voltage is output from the input buffer 121 and input into the voltage comparator 122, the output voltage of the voltage comparator 122 is off (becomes almost zero) from on.

Conventionally, the voltage comparison would be carried out by using an exclusive IC installed in the voltage comparator. In the present invention, in contrast, in order to simplify the electric circuit construction and realize the absolute voltage comparison, a Schmitt inverter (Schmitt circuit) as a digital IC is installed in the voltage comparator. In this case, therefore, the voltage comparator is utilized as an analog voltage comparator by using the threshold voltage of the Schmitt circuit as a reference voltage standard.

Generally, the Schmitt inverter is used in a digital circuit for realizing digital functions such as waveform shaping of digital waveform with noise. In this embodiment, the digital functions of the Schmitt inverter are utilized as analog functions in the voltage comparator 122. In this point of view, the difference between the threshold voltages when the voltage comparator is on from off and off from on is utilized to determine the standard voltage in analog. Therefore, the external controlling circuit cannot become unstable around the threshold voltage, thereby to be stabilized.

Moreover, since the threshold voltage of the Schmitt voltage corresponds to the reference standard voltage of the voltage comparator 122, the construction of the voltage comparator 122 can be simplified without an external standard voltage power supply and the operation of the voltage comparator 122 can be carried out stably and absolutely.

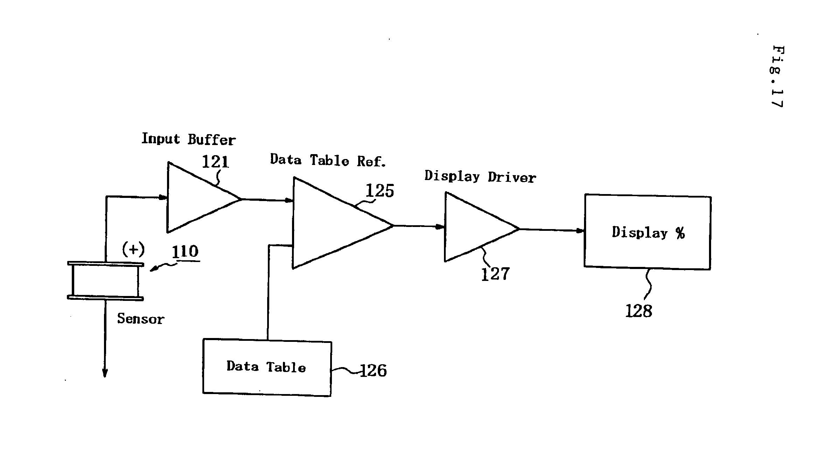

FIG. 17 is a block diagram of a hydrogen gas analyzer utilizing a hydrogen gas sensor according to the present invention.
In the hydrogen gas analyzer illustrated in FIG. 17, the electromotive force as a hydrogen gas detecting information from the hydrogen gas sensor 10 is converted in impedance and signal level by an input buffer 121 of higher input impedance, and input into a Data Table Reference circuit 25 provided at the next stage. In the Data Table Reference circuit 125 is input a Data Table 126 relating to the hydrogen gas concentrations and the electromotive forces of the hydrogen gas sensor, which the Data Table 126 is compared with an electromotive force input into the Data Table Reference circuit 125 to display the hydrogen gas concentration corresponding the input electromotive force via a Display Driver 127.

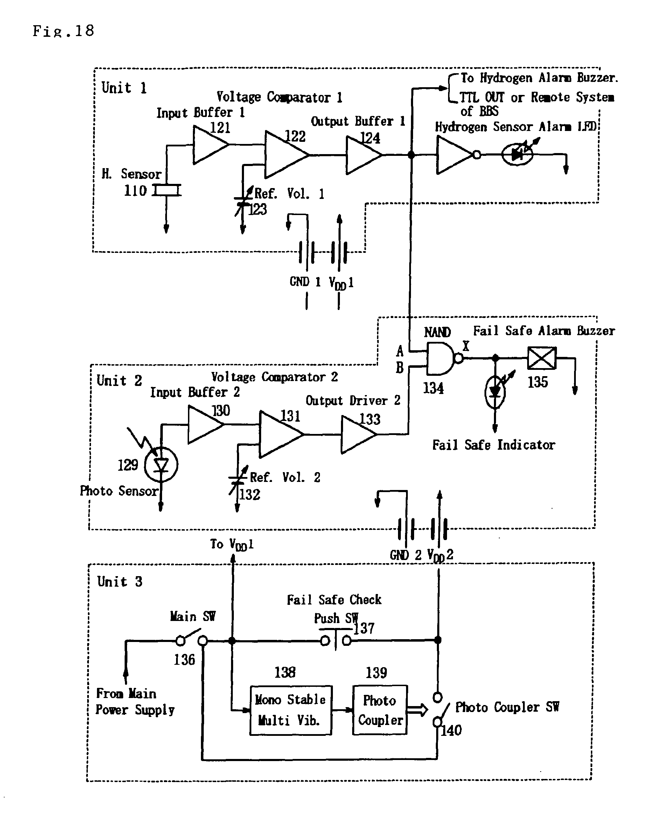

FIG. 18 is a block diagram of the hydrogen gas leak alarm system with Fail-Safe function. In this embodiment, the Fail-Safe functions (units 2 and 3) are combined with the hydrogen gas leak alarm system (unit 1) or the like. In the unit 1, the electromotive force variation as a hydrogen gas detecting information from the hydrogen gas sensor 110 is input into an input buffer 121 of high input impedance, converted in impedance and signal level, and input into a voltage comparator 122. In the voltage comparator 122, the input signal is compared with the reference voltage of a standard power supply 123, and the thus obtained compared result is output to a logical operating circuit 134 via an output buffer 124 provided at the next stage.

In the unit 2, the Fail-Safe function is applied to the hydrogen gas sensor element, the input buffer and the voltage comparator. A photo sensor 129 is installed in the hydrogen gas sensor element and monitors the contamination of the sensor component of the hydrogen gas sensor element introduced from external environment.

Information from the photo sensor 129 is input in an input buffer 130 of high input impedance, converted in impedance and signal level, and input in a voltage comparator 131. In the voltage comparator 131, the input signal is compared with the reference voltage of a standard power supply 132, and the thus obtained compared result is output to a logical operating circuit 134 via an Output Driver 133 provided at the next stage. In the logical operating circuit 134, the compared result relating to the input signal and the reference voltage is calculated, and an alarm buzzer 135 is switched off only when no hydrogen gas leak information is detected from the output buffer 24 and the operation of the photo sensor 129 is normal, that is, both of the output buffer 24 and the photo sensor 129 are stationary states. Except the stationary states of the output buffer 124 and the photo sensor 129, for example, when the hydrogen gas sensor 110 puts out a hydrogen gas detecting signal and/or the photo sensor 129 detects contamination from the external environment, the alarm buzzer 135 is switched on.

In the unit 3, the Fail-Safe function is applied to the light-emitting display for warning and the alarm buzzer. The operating conditions of the light-emitting display and the alarm buzzer are visually checked via a switch 37 for visual check or when the hydrogen gas leak alarm system is switched on.

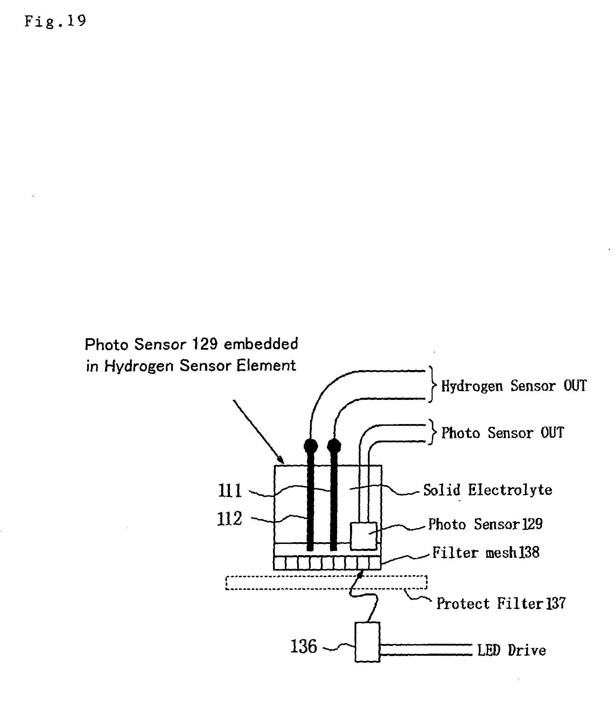

FIG. 19 is a schematic structural view of a hydrogen gas sensor element with Fail-Safe function. The Fail-Safe function relating to the hydrogen gas leak alarm system can be applied to the hydrogen gas sensor detecting section by calculating the signal from the hydrogen gas detecting section at the logical operating circuit 134 via the units 2 and 3. The Fail-Safe function can be also applied to the logical operating circuit 134 via another circuit, which is provided in parallel with the circuit 134.

In FIG. 19, the Fail-Safe function is applied to the hydrogen gas detecting section, and the LED signal from the LED 136 is transmitted via a protective mesh 137and detected at the photo sensor 129 via a translucent mesh 138. When the translucent mesh 138 is contaminated from external environment and thus, shut down, the signal from the photo sensor 129 is off, so that the photo sensor 129 is shut down against hydrogen gas due to the contamination. Since the hydrogen gas sensor element functions as an active element with spontaneous electromotive force under non-hydrogen gas atmosphere, the operating condition of the sensor element can be monitored by detecting the spontaneous electromotive voltage.

Although the present invention was described in detail with reference to the above examples, this invention is not limited to the above disclosure and every kind of variation and modification may be made without departing from the scope of the present invention.

### Brief Explanation of the Drawings:

FIG. 1 is a structural view for theoretically illustrating a hydrogen gas sensor according to the present invention;
FIG. 2 is a structural view illustrating a hydrogen gas sensor according to the embodiment of the present invention;
FIG. 3 is a graph showing a test result of the hydrogen gas sensor illustrated in FIG. 1;
FIG. 4 is a graph showing another test result of the hydrogen gas sensor illustrated in FIG. 1;
FIG. 5 is a structural view illustrating a modified embodiment of the hydrogen gas sensor illustrated in FIG. 2;
FIG. 6 is a structural view illustrating a modified embodiment of the hydrogen gas sensor illustrated in FIG. 5;
FIG. 7 is a structural view illustrating another modified embodiment of the hydrogen gas sensor illustrated in FIG. 2;
FIG. 8 is a structural view illustrating a hydrogen gas sensor according to another embodiment of the present invention;
FIG. 9 is a structural view illustrating a hydrogen gas sensor according to another embodiment of the present invention;
FIG. 10 is a view illustrating the state where the hydrogen gas sensor illustrated in FIG. 9 is integrated,
FIG. 11 is a structural view illustrating a hydrogen gas sensor array according to the present invention,
FIG. 12 is a view illustrating the state where a plurality of hydrogen gas sensors according to FIG. 11 are arranged and connected to one another in series,
FIG. 13 is a block diagram of a hydrogen gas leak alarming, controlling and information supply system utilizing a hydrogen gas sensor according to the present invention;
FIG. 14 is another block diagram of a hydrogen gas leak alarming, controlling and information supply system utilizing a hydrogen gas sensor according to the present invention;
FIG. 15 is a another block diagram of a hydrogen gas leak alarming, controlling and information supply system utilizing a hydrogen gas sensor according to the present invention;
FIG. 16 is a schematic explanatory view of the structure and operation of the voltage comparator in the systems illustrated in FIGS. 13-15;
FIG. 17 is a block diagram of a hydrogen gas analyzer utilizing a hydrogen gas sensor according to the present invention;
FIG. 18 is a block diagram of the hydrogen gas leak alarm system with Fail-Safe function; and
FIG. 19 is a schematic structural view of a hydrogen gas sensor element with Fail-Safe function.

### Brief Explanation of the Numerals:

- 10, 10A: hydrogen gas sensor
- 12, 12A: detecting electrode
- 14: standard electrode
- 16: electrolyte, solid electrolyte
- 18: electromotive force measuring device
- 20, 20a, 20b: envelope
- 20A: outer coating film
- 22: detecting surface
- 24: sealing portion
- 26: short-circuiting switch
- 28: controller
- 30: operative electrode
- 32: reinforcing casing
- 34: window
- 36: heater
- 38: absorbing agent
- 40: substrate
- 41: protective film

## Claims

1. A hydrogen gas sensor comprising a first electrode, a second electrode and an electrolyte contacting with said first electrode and said second electrode,
wherein said first electrode and said second electrode are made of corresponding different materials in chemical potential for hydrogen gas, and said first electrode is made of higher chemical potential material and said second electrode is made of lower chemical potential material,
wherein said hydrogen gas is detected on an electromotive force generated between said first electrode and said second electrode, and
wherein at least a contacting portion of said electrolyte with said first electrode and a contacting portion of said electrolyte with said second electrode are covered with a hydrogen selective permeable envelope.

2. The hydrogen gas sensor as defined in claim 1, wherein residual hydrogen gas within said envelope is reacted by temporary short-circuiting said pair of electrodes.

3. The hydrogen gas sensor as defined in claim 1 or 2, wherein said envelope is configured by superimposing multiple different materials having different barrier properties.

4. The hydrogen gas sensor as defined in any one of claims 1-3, further comprising a device for adjusting temperature of the space within said envelope.

5. The hydrogen gas sensor as defined in any one of claims 1-4,
wherein said first electrode is made of a hydrogen selective permeable metal.

6. A hydrogen gas leak alarm system comprising a hydrogen gas sensor as defined in any one of claims 1-5 and a voltage comparator,
wherein an electromotive force variation as a hydrogen gas detecting information from said hydrogen gas sensor is compared with a reference voltage of said voltage comparator, thereby to put out an signal on the comparison of said electromotive force variation and said reference voltage.

7. The hydrogen gas leak alarm system as defined in claim 6, wherein said voltage comparator is configured such that a threshold voltage of a Schmitt inverter is defined as said reference voltage, and compared with an input voltage corresponding to said hydrogen gas detecting information, thereby to put out said signal.

8. A hydrogen gas sensor array comprising a plurality of hydrogen gas sensors as defined in any one of claims 1-5, wherein said hydrogen gas sensors are arranged on the same substrate.

9. A hydrogen gas analyzer comprising a hydrogen gas sensor as defined in any one of claims 1-5 and an electric circuit for detecting an electromotive force from said hydrogen gas sensor,
wherein hydrogen gas concentration is detected in dependence on the intensity of said electromotive force.

10. A hydrogen gas sensor element comprising a hydrogen gas sensor as defined in any one of claims 1-5 and a photo sensor for detecting hydrogen gas shielding contamination from external environment through the detection of an optical signal from an external LED, whereby Fail-Safe function for enhancing reliability in hydrogen gas detection is applied to said hydrogen gas sensor element.
